# EUROPÄISCHE PATENTSCHRIFT

(11) **EP 1 493 463 B1**
(45) Veröffentlichungstag und Bekanntmachung des Hinweises auf die Patenterteilung: **04.04.2007**
(21) Anmeldenummer: 04090230.6
(22) Anmeldetag: 10.06.2004
(51) Int. Cl.: A61N 1/05

(54) **Elektrodenleitung**
Electrode lead
Fil d' electrode

(30) Priorität: 04.07.2003 DE 10331106
(43) Veröffentlichungstag der Anmeldung: 05.01.2005
(73) Patentinhaber: Biotronik GmbH & Co. KG, 12359 Berlin (DE)
(72) Erfinder: Kolberg, Gernot, 12043 Berlin (DE)
(74) Vertreter: Lindner-Vogt, Karin L.

(56) Entgegenhaltungen:
- EP-A- 0 653 223
- US-A- 5 951 597
- US-A- 5 954 761

## Beschreibung

Die Erfindung betrifft eine implantierbare Elektrodenleitung mit einem proximalen und einem distalen Ende, einer äußeren Elektrodenleitungsoberfläche und einer Elektrodenleitungslängsachse.

Bei Elektrodenleitungen, beispielsweise für Herzschrittmacher, stellt sich das Problem von Elektrodendislokalisationen. Bisherige Lösungen zur Fixierung von Elektrodenleitung im Herz und in Gefäßen sind beispielsweise vorgeformte Elektrodenleitungen, welche die Elektrode in Gefäßen verspannen. Auch bekannt sind am distalen Ende der Elektrodenleitung an der Elektrode angebrachte Schrauben oder Spiralen, welche ausgebildet sind, die Elektrode an ein Myokard durch Einschrauben zu fixieren. Weiter bekannt sind Ausformungen im distalen Bereich einer Elektrodenleitung, welche ausgebildet sind, die Elektrodenleitung im Trabekelwerk einer Herzkammer zu verankern.

US 5951597 zeigt eine Elektrodenleitung mit einem expandierbaren Befestigungskörper, der knapp proximal eines distalen Endes der Elektrodenleitung angeordnet ist.

Aus EP 0653223 ist eine Elektrodenleitung bekannt mit einer Vielzahl von Vorsprüngen oder Granulapartikeln am distalen Ende.

Aus dem Stand der Technik sind auch Haken bekannt, welche am distalen Ende oder auf der Elektrodenleitung angebracht sind und ausgebildet sind, die Elektrodenleitung am Myokard zu fixieren.

Die Patentanmeldung WO 97/31678 der Anmelderin beschreibt eine Elektrodenanordnung mit einer Ventrikelelektrode, welche hakenförmige Befestigungsmittel am distalen Ende der Elektrode aufweist.

Aus der DE 694 30 417 T2 ist ein Elektrodensystem bekannt, bei dem die Elektrodenleitung flexibel ausgebildet ist um zwischen der atrialen Elektrode und der ventrikulären Elektrode eine Schleife zu bilden. Diese Elektrodenleitung weist in der Nähe der atrialen Elektroden eine erste passive Befestigungsvorrichtung in Form von Haken, sowie in der Nähe der ventrikulären Elektroden am distalen Ende eine Befestigungsvorrichtung mit zwei Haken auf.

Alternativ zu diesem Stand der Technik besteht die der Erfindung zugrundeliegende Aufgabe darin, eine implantierbare Elektrodenleitung anzugeben, welche eine sichere Fixierung der Elektrodenleitung im Herz und in Gefäßen ermöglicht.

Diese Aufgabe wird durch eine Elektrodenleitung mit den Merkmalen des Patentanspruchs 1 gelöst. Vorteilhafte Ausführungsformen dieser Vorrichtung sind in den Unteransprüchen 2 bis 8 beschrieben.

Der Vorteil einer solchen Elektrodenleitung besteht darin, dass durch viele Mikrostrukturelemente, welche einen Bruchteil des Elektrodenleitungsdurchmessers messen und auf einen Oberflächenbereich der Elektrodenleitung verteilt sind, eine größere Haftung erzielt wird als beispielsweise bei einer Elektrodenleitung, welche nur einen oder einige Haken an der Oberfläche aufweist, um die Elektrodenleitung zu fixieren.

In einer bevorzugten Ausführungsform sind die Mikrostrukturelemente als Mikroborsten ausgebildet, wobei die Mikroborsten in einem vorbestimmten Winkel zur Elektrodenleitungsoberfläche angeordnet sind. Der Winkel kann beispielsweise 90° betragen. Dies bewirkt vorteilhaft, dass die Elektrodenleitung gegen eine längsaxiale Verschiebung sowohl in Richtung des proximalen Endes als auch in Richtung des distalen Endes gleichermaßen gesichert ist. In einer weiter bevorzugten Ausführungsform sind die Mikroborsten parallel zur Elektrodenleitungslängsachse angeordnet, besonders bevorzugt sind die Mikroborsten mit einer Richtungskomponente zum proximalen Ende der Elektrodenleitung weisend ausgerichtet. Das bewirkt vorteilhaft, dass die Elektrodenleitung sich bei der Implantation leicht einführen lässt und durch die Ausrichtung zum proximalen Ende hin ein erhöhter Widerstand gegen eine Verschiebung der Elektrodenleitung zum proximalen Ende gegeben ist. Alternativ zu dieser Ausführungsform kann ein mikrostrukturierter Oberflächenbereich sowohl Mikrostrukturelemente aufweisen, welche mit einer Richtungskomponente zum proximalen Ende weisend, als auch solche Mikrostrukturelemente, welche mit einer Richtungskomponente zum distalen Ende der Elektrodenleitung weisend ausgerichtet sind. Bevorzugt sind die Mikroborsten entlang der Elektrodenleitungslängsachse alternierend mit einer proximalen und einer distalen Richtungskomponente ausgerichtet. Das bewirkt vorteilhaft eine Fixierung der Elektrodenleitung bei einer Verschiebung entlang der Elektrodenleitungslängsachse sowohl in Richtung zum proximalen, als auch in Richtung zum distalen Ende der Elektrodenleitung.

In einer anderen Ausführungsvariante sind die Mikroborsten in einem vorbestimmten Winkel zur Elektrodenleitungsoberfläche angeordnet und in einem vorbestimmten Winkel zur Längsachse der Elektrodenleitung jeweils parallel zueinander ausgerichtet. Bevorzugt sind die Mikroborsten in dieser Ausführungsvariante mit einer Richtungskomponente zum proximalen Ende der Elektrodenleitung weisend ausgerichtet. Das bewirkt vorteilhaft, dass die Elektrodenleitung durch eine Drehung um die Längsachse in Umfangsrichtung in ein Gefäß einschraubbar ist. Bevorzugt sind die Borsten ausgebildet, sich bei dem Einschrauben flach an die Elektrodenoberfläche anzulegen. Eine Drehung entgegengesetzt zur Einschraubrichtung bewirkt eine Fixierung der Elektrodenleitung, eine wiederholte Drehung in Einschraubrichtung, ein Lösen der Elektrodenleitung.

In einer anderen Ausführung sind die Mikrostrukturelemente als Schuppen mit einer Schuppenkante ausgebildet. Bevorzugt sind die Schuppen in einem vorbestimmten Winkel, weiter bevorzugt spitzen Winkel zur Elektrodenleitungsoberfläche derart angeordnet, dass die Schuppenkante von der Elektrodenleitungsoberfläche beabstandet ist. Weiter bevorzugt sind die Schuppen derart angeordnet, dass wenigstens ein Kantenabschnitt der Schuppenkante eine senkrecht zur Elektrodenleitungslängsachse verlaufende Ausrichtungskomponente aufweist. Das bewirkt vorteilhaft, dass die Elektrodenleitung bei Berührung mit einer Gefäßwand entlang der Elektrodenleitungslängsachse in einer Richtung leicht verschieblich ist und in der dazu entgegengesetzten Richtung durch Eingreifen der Schuppenkanten in die Gefäßwand ein erhöhter Widerstand gegen eine längsaxiale Verschiebung gegeben ist.

In einer weiter bevorzugten Ausführungsform weisen die Schuppen an der Schuppenkante wenigstens eine Zacke auf. Diese Zacke kann vorteilhaft zusätzlich zu der Schuppenkante in die Gefäßwand eingreifen und somit einen weiter erhöhten Widerstand gegen eine längsaxiale Verschiebung ausbilden.

Die Mikrostrukturelemente können auf die Elektrodenleitungsoberfläche aufgebracht oder aus dieser ausgeformt sein. Bevorzugt ist die Mikrostruktur in einem mikrostereolithografischen Verfahren hergestellt, beispielsweise durch Abscheiden auf einem Substrat. Das Substrat kann auf der Elektrodenleitungsoberfläche aufgebracht sein oder durch die Elektrodenleitungsoberfläche selbst gebildet sein. Alternativ kann ein Oberflächenbereich mit einer Mikrostruktur auch durch eine mikrostereolithografisch erzeugte Form hergestellt sein.

Eine Elektrodenleitung kann einen oder mehrere Oberflächenbereiche mit einer Mikrostruktur aufweisen, wobei eine Ausführungsform oder eine Kombination verschiedener Ausführungsformen von Mikrostrukturelementen wie beispielsweise Mikroborsten, Schuppen, Noppen oder die in den Figuren dargestellten Ausführungsformen von Mikrostrukturelementen, auf genau einer Elektrodenleitung verwirklicht sein kann.

Geeignete Materialien für die Elektrodenleitung sind beispielsweise Poly-Tetra-Fluor-Ethylen (PTFE), Silikone, Polyimid, Ethylenchlortrifluorethylen, Ethylentetraflourethylen (ETFE), Polypropylen oder Polyurethane. Die Mikrostrukturen können gemeinsam mit der Elektrodenleitung oder einem Abschnitt der Elektrodenleitung im Spritzgussverfahren hergestellt werden, wobei die Mikrostrukturen in einer Spritzgussform entsprechend eingearbeitet sein können.

Zum leichten Einführen der Elektrodenleitung in ein Lumen kann eine Elektrodenleitung eine längsaxial verschiebliche Hülle aufweisen, welche sich auf der Elektrodenleitung derart anordnen lässt, die Mikrostruktur zu verdecken und im Anwendungsfall durch Zurückziehen in proximale Richtung die Mikrostruktur freizugeben.

Die Erfindung soll nun anhand der Figuren näher erläutert werden.
- Figur 1: zeigt das distale Ende einer Elektrodenleitung mit einer Elektrodenleitungsoberfläche und Oberflächenbereichen, welche jeweils eine Mikrostruktur aufweisen;
- Figur 2: zeigt verschiedene Ausführungsformen von Mikrostrukturelementen;
- Figur 3: zeigt ein Anordnungsbeispiel von Mikroborsten auf einer Elektrodenleitung.
- Figur 4: zeigt Abschnitte einer Elektrodenleitung mit verschiedenen Ausführungsformen von Mikrostrukturelementen, welche als Schuppen ausgebildet sind;
- Figur 5: zeigt schematisiert dargestellte Anordnungen von Noppen, die nicht gemäß der Erfindung sind;
- Figur 6: zeigt ein Ausführungsbeispiel, in welchem ein Elektrodenkopf mit dem distalen Ende einer Elektrodenleitung über eine Verbindungsleitung schnurverbunden ist.

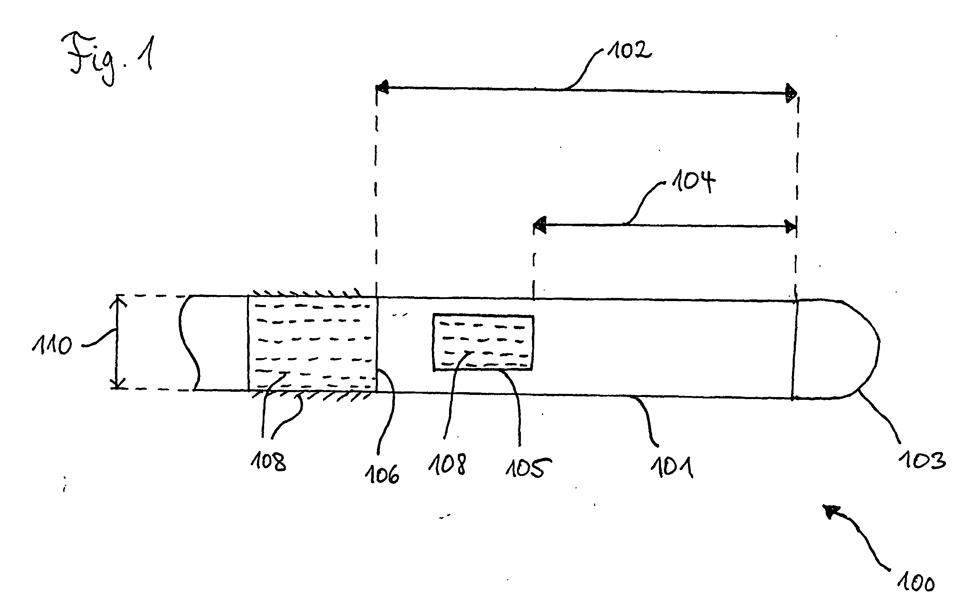

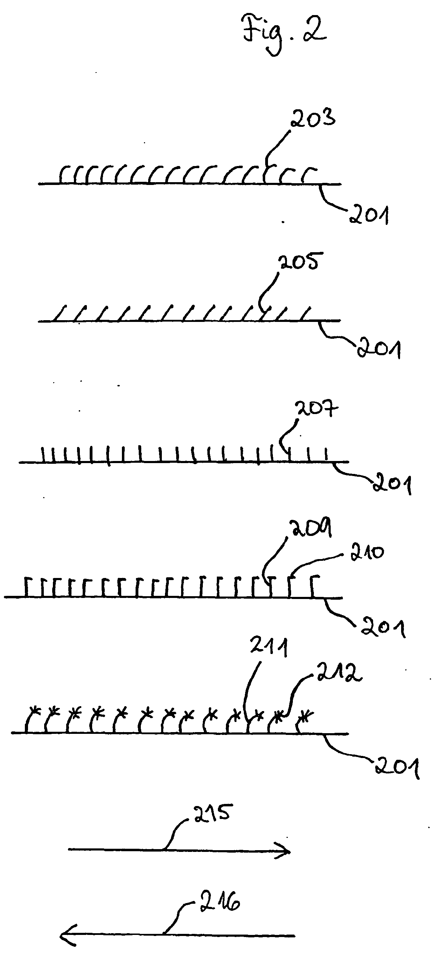

Figur 1 zeigt - schematisch dargestellt - das distale Ende einer Elektrodenleitung 100 mit einer Elektrodenleitungsoberfläche 101 und Oberflächenbereichen 105 und 106, welche jeweils eine Mikrostruktur aufweisen. Der Oberflächenbereich 105 ist im Abstand 104 von einer am distalen Ende der Elektrodenleitung befindlichen Elektrode 103 angeordnet. Der Oberflächenbereich 105 mit einer Mikrostruktur belegt in Umfangsrichtung nur einen Teil der Elektrodenleitungsoberfläche. In diesem Beispiel ist auch ein umlaufender Oberflächenbereich 106 dargestellt, welcher in voller Umfangsrichtung die äußere Elektrodenleitungsoberfläche 101 bedeckt. Der Oberflächenbereich 106 ist in diesem Beispiel im Abstand 102 von der am distalen Ende befindlichen Elektrode 103 angeordnet. In dieser Figur sind zwei der Ausführungsvarianten von Oberflächenbereichen - umlaufend und nicht umlaufend - gemeinsam auf einer Elektrodenleitung dargestellt. Eine Elektrodenleitung im Sinne der Erfindung kann mindestens einen in Umfangsrichtung der Elektrodenleitung umlaufenden Oberflächenbereich 106 oder mindestens einen nicht umlaufenden Oberflächenbereich 105 aufweisen. Alternativ dazu kann eine Elektrodenleitung im Sinne der Erfindung mehrere in Umfangsrichtung der Elektrodenleitung umlaufende Oberflächenbereiche 106 oder mehrere nicht umlaufende Oberflächenbereiche 105 aufweisen. Auch eine Kombination von umlaufenden Oberflächenbereichen 106 und nicht umlaufenden Oberflächenbereichen 105 auf einer Elektrodenleitung ergeben verschiedene, hier nicht abgebildete Ausführungsvarianten. Die Abstände 102 und 104 können beide mehrere Elektrodenleitungsdurchmesser 110 betragen und sind in diesem Beispiel schematisch dargestellt. Die Oberflächenbereiche 106 und 105 weisen eine Mikrostruktur auf, wobei die Mikrostruktur durch eine Vielzahl von auf der Elektrodenleitungsoberfläche angeordneten, über den Oberflächenbereich sowohl in Längsrichtung als auch in Umfangsrichtung verteilten Mikrostrukturelementen gebildet ist. Verschiedene Ausführungsformen von Mikrostrukturelementen werden im Folgenden in Figur 2 dargestellt.

Figur 2 zeigt eine schematische Darstellung verschiedener Ausführungsformen von Mikrostrukturelementen. Diese Mikrostrukturelemente haben eine radiale Ausdehnung über die Elektrodenleitungsoberfläche hinaus, welche einen Bruchteil des Elektrodenleitungsdurchmessers beträgt. Die Mikrostrukturelemente sind ausgebildet, bei Berühren einer Gefäßwand einer Verschiebung der Elektrodenleitung in Längsrichtung entgegenzuwirken.

Auf der Elektrodenleitungsoberfläche 201 sind beispielsweise Mikrostrukturelemente 203 angebracht, welche als gekrümmte Mikroborsten ausgebildet sind. Die Mikroborsten 203 sind mit einer in proximale Richtung 215 weisenden Richtungskomponente gekrümmt. Die Mikroborsten 203 erzeugen dadurch bei Verschiebung der Elektrodenleitung in proximale Richtung 215 einen erhöhten Widerstand gegenüber einer Verschiebung der Elektrodenleitung in distale Richtung 216.

Die Mikroborsten können auch als geradlinige Mikroborsten 205 ausgebildet sein. Diese geradlinig ausgebildeten Mikroborsten 205 sind in einem vorbestimmten Winkel zur Elektrodenleitungsoberfläche 201 derart angeordnet, dass sie eine Ausrichtungskomponente in proximale Richtung 215 aufweisen.

Die Mikroborsten 207 sind ebenfalls geradlinig ausgebildet und sind senkrecht auf der Elektrodenleitungsoberfläche 201 angeordnet. Dadurch wird bei einer Verschiebung der Elektrodenleitung in proximale Richtung 215 und in distale Richtung 216 ein gleichermaßen hoher Widerstand erzeugt.

Die Mikroborsten 209 sind auf der Elektrodenleitungsoberfläche 201 angebracht und weisen an dem der Elektrodenleitungsoberfläche 201 abgewandten Ende einen in proximale Richtung 215 angeformten Haken 210 auf. Dieser Haken 210 ist ausgebildet, bei einer Verschiebung der Elektrodenleitung in proximale Richtung 215 in eine Gefäßwand einzugreifen und dadurch einen besonders hohen Widerstand gegen eine Verschiebung der Elektrodenleitung in proximale Richtung 215 zu erzeugen.

Die Mikroborsten 211 sind ebenfalls wie die Mikroborsten 203 in proximale Richtung 215 gekrümmt und weisen an dem zur Elektrodenleitungsoberfläche 201 abgewandten Ende eine sternförmige Verästelung 212 auf. Durch diese Ausbildung können die Mikroborsten 211 bei Berührung mit einer Gefäßwand besonders effektiv in diese Eingreifen und erzeugen damit einen hohen Widerstand gegen eine Verschiebung der Elektrodenleitung in proximale Richtung 215.

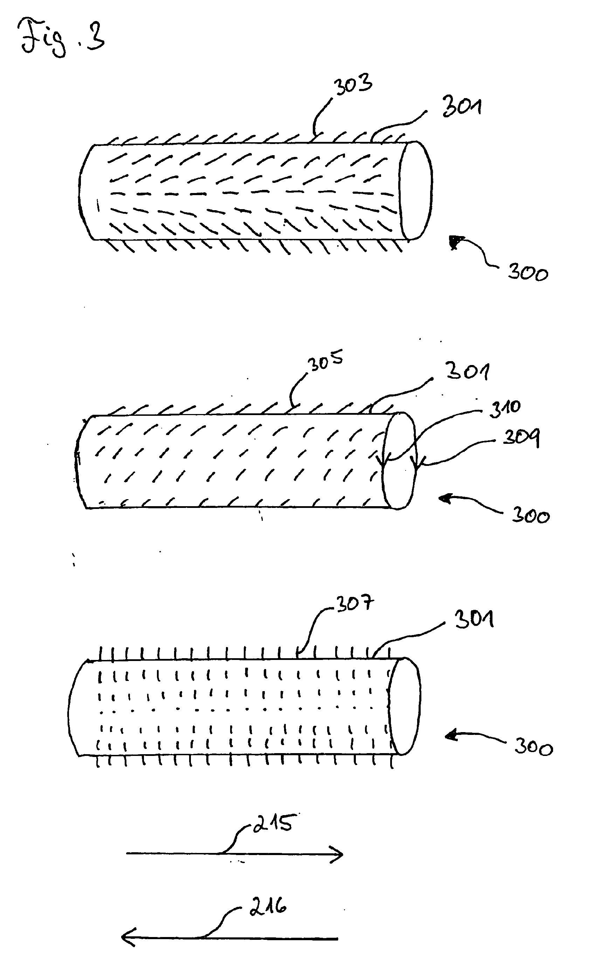

Figur 3 zeigt schematisch dargestellt ein Anordnungsbeispiel von Mikroborsten auf einer Elektrodenleitung. Dargestellt sind Abschnitte einer Elektrodenleitung 300, auf deren Elektrodenleitungsoberfläche 301 Mikroborsten angebracht sind. Die Mikroborsten 303 sind in einem vorbestimmten Winkel zur Elektrodenleitungsoberfläche 301 mit einer Richtungskomponente in proximale Richtung 215 ausgerichtet.

In einem weiteren Beispiel sind Mikroborsten 305 in einem vorbestimmten Winkel zur Elektrodenleitungsoberfläche 301 angeordnet und mit einer Richtungskomponente in proximale Richtung 215 ausgerichtet. Die Mikroborsten 305 sind auch in einem vorbestimmten Winkel zur Elektrodenleitungslängsachse jeweils parallel zueinander ausgerichtet, so dass bei einer Drehung der Elektrodenleitung in die Umfangsrichtung 309 sich die Mikroborsten 305 bei Berührung einer Gefäßwand in dieser verankern. Bei Drehung der Elektrodenleitung in der zur Umfangsrichtung 309 entgegengesetzten Richtung 310 können die Mikroborsten 305 sich aus der Gefäßwandverankerung wieder lösen.

Die Mikroborsten 307 sind orthogonal auf der Elektrodenleitungsoberfläche 301 angeordnet. Dadurch wird sowohl bei Verschiebung der Elektrodenleitung in proximale Richtung 215 als auch bei Verschiebung der Elektrodenleitung in distale Richtung 216 ein gleichermaßen hoher Widerstand der Verschiebungsbewegung entgegen gesetzt.

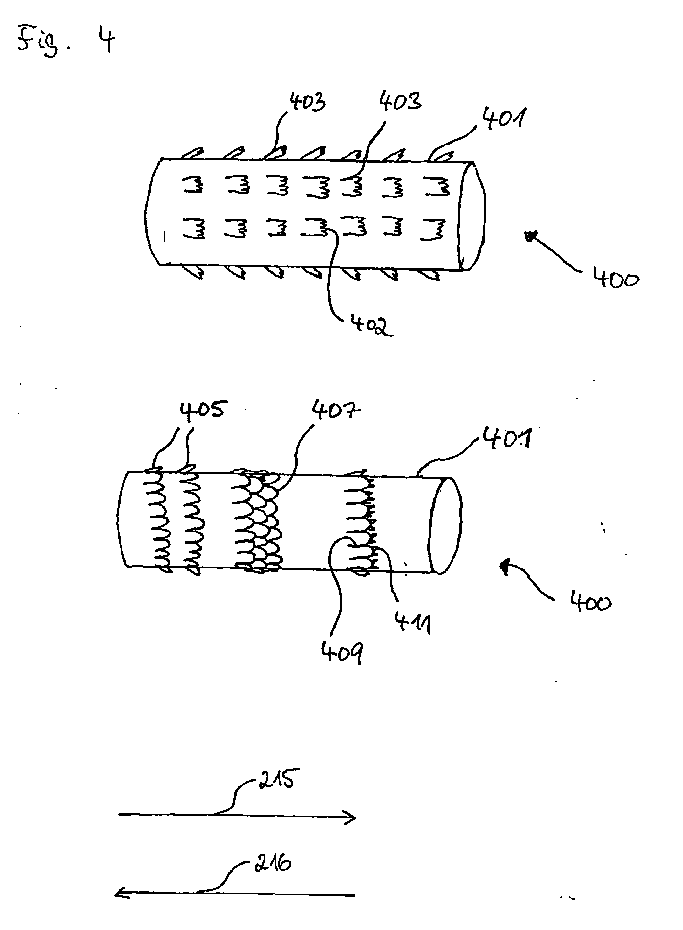

Figur 4 zeigt schematisch dargestellt Abschnitte einer Elektrodenleitung 400 mit verschiedenen Ausführungsformen von Mikrostrukturelementen, welche als Schuppen ausgebildet sind. Die in dieser Figur gezeigten Ausführungsformen sind teilweise gemeinsam auf einem Elektrodenleitungsabschnitt dargestellt. Eine Elektrodenleitung kann jeweils eine der gezeigten Ausführungsvarianten oder eine Kombination verschiedener Ausführungsvarianten aufweisen.

Die Schuppen 403 sind an einem vorbestimmten Winkel zur Elektrodenleitungsoberfläche 401 in proximale Richtung 215 ausgerichtet. Die Schuppen 403 weisen eine senkrecht zur Elektrodenleitungslängsachse verlaufende Kante auf, welche eine Verzahnung 402 aufweist. Diese Verzahnung ist ausgebildet, bei einer Verschiebung der Elektrodenleitung in proximale Richtung 215 besonders effektiv in eine Gefäßwand einzugreifen und sich in dieser zu verankern.

In einem weiteren Beispiel sind Schuppen 405 mit einer runden Kante nebeneinander umlaufend in Umfangsrichtung einer Elektrodenleitung angeordnet. Die Schuppen 405 bilden Ringe auf der Elektrodenleitungsoberfläche 401, welche voneinander beabstandet sind. Weiter rechts auf diesem Abschnitt ist ein Ausführungsbeispiel dargestellt, in welchem Schuppen 407 mit einer abgerundeten Kante derart angeordnet sind, dass längsaxial aufeinanderfolgende Schuppen jeweils um eine halbe Schuppenbreite in Umfangsrichtung zueinander versetzt sind. Weiter sind auf dem Abschnitt in Umfangsrichtung Schuppen 409 mit einer runden Schuppenkante nebeneinander angeordnet, wobei die Schuppenkante an mindestens einem in Umfangsrichtung verlaufenden Kantenabschnitt in proximale Richtung 215 ausgerichtete Zacken aufweist. Nicht gezeigte Ausführungsvarianten einer Elektrodenleitung können jeweils eine der Schuppenvarianten 405, 407 oder 409 umfassen. Auch eine Kombination der Schuppenvariante 409 mit der Schuppenvariante 407 auf einer Elektrodenleitung oder eine Kombination der Schuppenvariante 409 mit der Schuppenvariante 405 auf einer Elektrodenleitung sind mögliche Ausführungsvarianten.

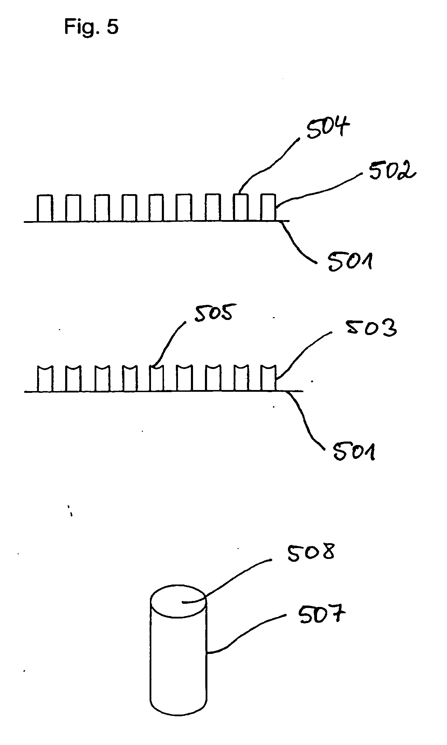

In Figur 5 sind Anordnungen von Noppen schematisiert dargestellt. Die Noppenanordnungen 502 und 503 sind auf einer Elektrodenleitungsoberfläche 501 angebracht und in diesem Beispiel in einer Seitenansicht als Schnittbild schematisiert dargestellt.

Die Noppe 507 ist als Zylinderabschnitt ausgeformt und weist eine kreisförmige Adhäsionsfläche 508 auf, welche ausgebildet ist, durch Adhäsion an einer Gefäßwand zu haften.

Die Noppen 502 und 503 können wie die Noppe 507 zylindrisch ausgeformt sein. Bei der Noppenform 502 ist die Adhäsionsfläche 504 eben ausgebildet. Bei der Noppe 503 ist die Adhäsionsfläche 505 konkav ausgeformt und somit als Saugnapf ausgebildet.

In einer nicht dargestellten Anordnungsvariante kann alternativ zu der Kreisform die Adhäsionsfläche auch als Polygon ausgebildet sein.

Eine Elektrodenleitung in Sinne der Erfindung kann eine Kombination aus umlaufenden oder nicht umlaufenden Oberflächenbereichen mit jeweils einer beliebigen Kombination der zuvor aufgezeigten Ausführungsvarianten von als Mikroborsten, Schuppen, oder Noppen ausgebildeten Mikrostrukturelementen aufweisen.

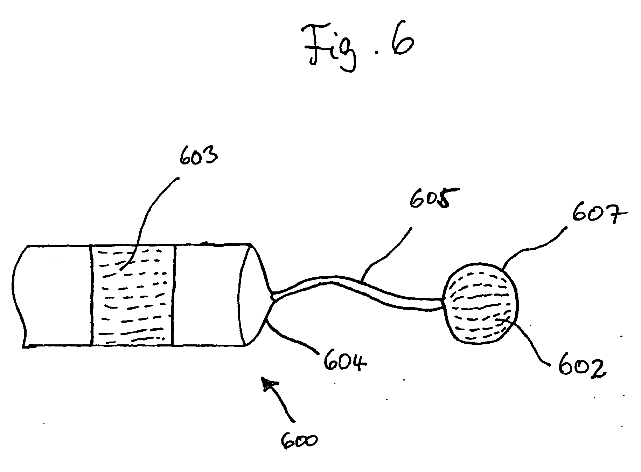

Eine Weiterbildung der Erfindung ist in Figur 6 schematisch dargestellt. In diesem Ausführungsbeispiel ist ein Elektrodenkopf 607 mit dem distalen Ende einer Elektrodenleitung über eine Verbindungsleitung 605 schnurverbunden. Im Bereich des distalen Endes 600 weist die Elektrodenleitung einen Oberflächenbereich 603 mit einer Mikrostruktur auf. Der Oberflächenbereich 603 ist in diesem Beispiel umlaufend in Umfangsrichtung ausgebildet. In diesem Ausführungsbeispiel ist die Verbindungsleitung 605 mit einer Dichtungsmasse 604, beispielsweise eine Silikon-Dichtungsmasse, gegen den Elektrodenleitungskörper am distalen Ende abgedichtet. Sowohl die Elektrode 607 als auch der Oberflächenbereich 603 am distalen Ende 600 der Elektrodenleitung weisen eine Mikrostruktur 603 auf, welche durch Mikrostrukturelemente gebildet ist. Die Mikrostrukturelemente der Mikrostruktur 602 sind bevorzugt elektrisch leitfähig ausgebildet, beispielsweise kann der Elektrodenkopf 607 das Elektrodenleitungsmaterial enthalten und eine Gold- oder Platinschicht an der Oberfläche aufweisen. Die Gold- oder Platinschicht kann aufgedampft oder aufgesputtert sein. Diese Ausführungsform gestattet vorteilhaft eine Relativbewegung entlang der Elektrodenleitungslängsachse zwischen dem Elektrodenkopf 607 und dem distalen Ende 600 der Elektrodenleitung. Die Mikrostrukturelemente der Mikrostrukturen 602 und 603 können als Mikroborsten oder Noppen in einer oder einer Kombination der zuvor beschriebenen Ausführungsformen und Anordnungen verwirklicht sein. In einer nicht dargestellten alternativen Ausführungsform weist die Elektrodenleitung analog zu Figur 6 einen Elektrodenkopf auf, welcher keine Mikrostruktur aufweist. Der Elektrodenkopf ist somit als eine frei schwimmende Elektrode, bevorzugt als Ventrikelelektrode, ausgebildet.

In einer Weiterbildung der Erfindung ist die Mikrostruktur des Oberflächenbereiches der Elektrodenleitung derart ausgebildet, dass Körpergewebe in den Oberflächenbereich einwachsen kann.

## Patentansprüche

1. Implantierbare Elektrodenleitung mit einem proximalen und einem distalen Ende, einer äußeren Elektrodenleitungsoberfläche und einer Elektrodenleitungslängsachse,
wobei
die Elektrodenleitung im Bereich des distalen Endes einen zur seitlichen Berührung einer Gefäßwand vorgesehenen, in einem mehrere Elektrodenleitungsdurchmesser messenden Abstand zum distalen Ende angeordneten Oberflächenbereich mit einer Mikrostruktur aufweist, wobei die Mikrostruktur durch eine Vielzahl von auf der Elektrodenleitungsoberfläche angeordneten, über dem Oberflächenbereich sowohl in Längsrichtung als auch in Umfangsrichtung verteilten, Mikrostrukturelementen mit einer radialen Erstreckungskomponente gebildet ist; wobei die Mikrostrukturelemente eine radiale Ausdehnung haben, welche einen Bruchteil des Elektrodenleitungsdurchmessers beträgt und ausgebildet sind, bei Berühren einer Gefäßwand einer Verschiebung der Elektrodenleitung entlang der Elektrodenleitungslängsachse entgegenzuwirken, **dadurch gekennzeichnet,**
**dass** die Mikrostrukturelemente aufweden als Mikroborsten ausgebildet sind, wobei die Mikroborsten in einem vorbestimmten Winkel zur Elektrodenleitungsoberfläche ausgerichtet sind, oder als Schuppen mit einer Schuppenkante ausgebildet sind.

2. Implantierbare Elektrodenleitung nach Anspruch 1, **dadurch gekennzeichnet, dass** die Mikroborsten in einem vorbestimmten Winkel zur Elektrodenleitungsoberfläche angeordnet sind und in einem vorbestimmten Winkel zur Elektrodenleitungslängsachse jeweils parallel zueinander ausgerichtet sind.

3. Implantierbare Elektrodenleitung nach Anspruch 1 bis 2, **dadurch gekennzeichnet, dass** die Mikroborsten mit einer Richtungskomponente zum proximalen Ende der Elektrodenleitung weisend ausgerichtet sind.

4. Implantierbare Elektrodenleitung nach Anspruch 1, **dadurch gekennzeichnet, dass** die Schuppen in einem vorbestimmten Winkel zur Elektrodenleitungsoberfläche derart angeordnet sind, dass die Schuppen von der Elektrodenleitungsoberfläche abstehen.

5. Implantierbare Elektrodenleitung nach Anspruch 4, **dadurch gekennzeichnet, dass** die Schuppen derart angeordnet sind, dass wenigstens ein Kantenabschnitt der Schuppenkante eine senkrecht zur Elektrodenleitungslängsachse verlaufende Ausrichtungskomponente aufweist.

6. Implantierbare Elektrodenleitung nach einem der Ansprüche 1, 4 oder 5, **dadurch gekennzeichnet, dass** die Schuppen an der Schuppenkante wenigstens eine Zacke aufweisen.

7. Implantierbare Elektrodenleitung nach einem der vorhergehenden Ansprüche, **dadurch gekennzeichnet, dass** die Mikrostrukturelemente aus der Elektrodenleitungsoberfläche ausgeformt sind.

8. Implantierbare Elektrodenleitung nach einem der vorhergehenden Ansprüche, **dadurch gekennzeichnet, dass** die Mikrostrukturelemente auf der Elektrodenleitungsoberfläche aufgebracht sind.

## Claims

1. An implantable electrode line with a proximal and distal end, an outer electrode line surface and a longitudinal electrode line axis, wherein the electrode line, in the region of the distal end, has a surface region with a microstructure provided for lateral contact with a vessel wall and arranged at a distance to the distal end measuring a plurality of electrode line diameters, wherein the microstructure is formed by a multiplicity of microstructural elements with a radial extension component arranged on the electrode line surface and distributed over the surface region both in the longitudinal and circumferential direction, wherein the microstructural elements have a radial extension which is a fraction of the electrode line diameter, and are designed to counteract a displacement of the electrode line along the longitudinal electrode line axis on contact with a vessel wall, **characterised in that** the microstructural elements are designed either as micro-bristles, wherein the micro-bristles are aligned at a predetermined angle to the electrode line surface, or are designed as scallops with a scalloped edge.

2. The implantable electrode line according to Claim 1, **characterised in that** the micro-bristles are arranged at a predetermined angle to the electrode line surface and are each aligned parallel with each other at a predetermined angle to the longitudinal electrode line axis.

3. The implantable electrode line according to Claim 1 or 2, **characterised in that** the micro-bristles are aligned with a direction component pointing to the proximal end of the electrode line.

4. The implantable electrode line according to Claim 1, **characterised in that** the scallops are arranged at a predetermined angle to the electrode line surface so that the scallops project from the electrode line surface.

5. The implantable electrode line according to Claim 4, **characterised in that** the scallops are arranged so that at least one edge section of the scalloped edge has an alignment component running perpendicularly to the longitudinal electrode line axis.

6. The implantable electrode line according to one of Claims 1, 4 or 5, **characterised in that** the scallops on the scalloped edge have at least one indentation.

7. The implantable electrode line according to one of the preceding claims, **characterised in that** the microstructural elements are formed from the electrode line surface.

8. The implantable electrode line according to one of the preceding claims, **characterised in that** the microstructural elements are fitted on the electrode line surface.

## Revendications

1. Ligne d'électrodes implantable comprenant une extrémité proximale et une extrémité distale, une surface extérieure de ligne d'électrodes et un axe longitudinal de ligne d'électrodes, la ligne d'électrodes présentant dans la zone de l'extrémité distale une zone de surface, prévue pour le contact latéral avec une paroi de vaisseau, disposée à une distance mesurant plusieurs diamètres de ligne d'électrodes à l'extrémité distale, avec une microstructure, la microstructure étant formée par une pluralité d'éléments de microstructure, disposés sur la surface de la ligne d'électrodes et répartis sur la zone de surface aussi bien dans le sens longitudinal que dans le sens périphérique, avec une composante d'extension radiale, les éléments de microstructure ayant une extension radiale qui représente une fraction du diamètre de la ligne d'électrodes, et étant conçus pour s'opposer à un déplacement de la ligne d'électrodes le long de l'axe longitudinal de la ligne d'électrodes en cas de contact avec une paroi de vaisseau, **caractérisée en ce que** les éléments de microstructure sont conçus soit sous la forme de microcrins, les microcrins étant orientés dans un angle prédéfini par rapport à la surface de ligne d'électrodes, soit en forme d'écailles avec une arête d'écaille.

2. Ligne d'électrodes implantable selon la revendication 1, **caractérisée en ce que** les microcrins sont disposés dans un angle prédéfini par rapport à la surface de ligne d'électrodes et sont orientés parallèlement les uns par rapport aux autres dans un angle prédéfini par rapport à l'axe longitudinal de la ligne d'électrodes.

3. Ligne d'électrodes implantable selon les revendications 1 à 2, **caractérisée en ce que** les microcrins sont orientés avec une composante de direction dirigée vers l'extrémité proximale de la ligne d'électrodes.

4. Ligne d'électrodes implantable selon la revendication 1, **caractérisée en ce que** les écailles sont disposées dans un angle prédéfini par rapport à la surface de ligne d'électrodes de telle sorte que les écailles dépassent de la surface de ligne d'électrodes.

5. Ligne d'électrodes implantable selon la revendication 4, **caractérisée en ce que** les écailles sont disposées de telle sorte qu'au moins une partie de l'arête d'écaille présente une composante d'orientation agencée perpendiculairement à l'axe longitudinal de la ligne d'électrodes.

6. Ligne d'électrodes implantable selon l'une quelconque des revendications 1, 4 ou 5, **caractérisée en ce que** les écailles présentent au moins une pointe sur l'arête d'écaille.

7. Ligne d'électrodes implantable selon l'une quelconque des revendications précédentes, **caractérisée en ce que** les éléments de microstructure sont formés à partir de la surface de ligne d'électrodes.

8. Ligne d'électrodes implantable selon l'une quelconque des revendications précédentes, **caractérisée en ce que** les éléments de microstructure sont appliqués sur la surface de ligne d'électrodes.
